# FASCICULE DE BREVET EUROPEEN

(11) **EP 1 449 478 B1**
(45) Date de publication et mention de la délivrance du brevet: **27.10.2010**
(21) Numéro de dépôt: 04290221.3
(22) Date de dépôt: 28.01.2004
(51) Int. Cl.: A61B 5/15, A61M 1/02

(54) **Sytème à poches comprenant un moyen d'association de récipients d'échantillonnage**
Beutelsystem mit einem Anschluss für einen Probenentnahmebehälter
Multiple bag system comprising a connector for a probe withdrawal container

(30) Priorité: 19.02.2003 FR 0302034
(43) Date de publication de la demande: 25.08.2004
(73) Titulaire: MACO PHARMA, 59420 Mouvaux (FR)
(72) Inventeur: Demay, Sylvie, 59250 Halluin (FR); Goudaliez, Francis, 59155 Faches-Thumesnil (FR); Verpoort, Thierry, 59420 Mouvaux (FR)
(74) Mandataire: Novagraaf Technologies

(56) Documents cités:
- EP-A- 0 510 615
- EP-A- 0 678 279
- EP-A- 1 136 087
- WO-A-01/08546
- DE-A- 4 120 267
- DE-C- 10 117 726
- US-A- 3 528 404
- US-A- 5 871 699

## Description

L'invention concerne un système à poches pour le prélèvement d'un fluide biologique.

Elle s'applique typiquement au cas où le fluide biologique est du sang total qui doit être prélevé d'un donneur dans une poche de recueil. Pour ce faire, le système à poches comprend, en circuit clos, un dispositif de prélèvement du sang qui est en communication fluidique avec au moins une poche de recueil du sang. En outre, le système comprend un dispositif d'échantillonnage du sang qui est destiné à recevoir une partie du sang prélevé, ledit dispositif comprenant au moins un récipient d'échantillonnage.

L'utilisation d'un tel dispositif d'échantillonnage permet d'obtenir, dans chaque récipient, un échantillon de sang destiné à être analysé, notamment pour réaliser une sérologie, une virologie et une numération.

En particulier, le système à poches peut être utilisé en prélevant les premiers millilitres de sang dans le dispositif d'échantillonnage, ce qui présente un certain nombre d'avantages. Premièrement, cela permet de diminuer le risque de contamination provenant de la présence de bactéries ou d'autres substances étrangères sur la peau du donneur, puisque les premiers millilitres de sang prélevés concernés par cette contamination sont envoyés dans le dispositif d'échantillonnage et non dans la poche de recueil. Deuxièmement, cela permet de réaliser les échantillons avant que la poche ne soit totalement remplie, et par conséquent de ne pas perdre de temps. Enfin, lors du prélèvement, la perte de volume de sang pour le donneur étant compensée par l'apport de plasma, l'hématocrite du sang à analyser serait plus bas si le dispositif d'échantillonnage était rempli après la poche de recueil, et par conséquent la numération serait faussée.

Un problème qui se pose est celui de la difficulté de manipulation des systèmes à poches connus.

En effet, l'utilisateur doit mettre en place plusieurs récipients pour obtenir les échantillons ce qui est générateur de perte de temps.

L'invention a notamment pour but de résoudre ce problème en proposant un système à poches dans lequel chaque récipient d'échantillonnage est stocké au niveau du moyen de transfert du fluide depuis le système à poches dans celui-ci, ledit récipient pouvant ensuite être guidé par l'opérateur dans le moyen de transfert pour la prise d'échantillon.

A cet effet, l'invention concerne un système à poches pour le prélèvement d'un fluide biologique, comme défini dans la revendication 1.

D'autres objets et avantages de l'invention apparaîtront au cours de la description qui suit, faite en référence aux dessins annexés dans lesquels :
- la figure 1a représente de façon schématique un système à poches pour le prélèvement du sang qui comprend un dispositif d'échantillonnage selon un premier mode de réalisation ;
- la figure 1b représente de façon schématique un système à poches pour le prélèvement du sang et la séparation des composants sanguins qui comprend un dispositif d'échantillonnage selon un deuxième mode de réalisation ;
- la figure 2 représente de façon schématique le moyen de transfert du dispositif d'échantillonnage représenté sur la figure 1a ;
- les figures 3a et 3b représentent de façon schématique le moyen de transfert de la figure 2 dans lequel un récipient d'échantillonnage est disposé respectivement dans une position à distance et dans une position de transfert ; la figure 3c est une représentation analogue à la figure 3b montrant une variante de réalisation du moyen de transfert ;
- la figure 4 représente de façon schématique un système à poches pour le prélèvement du sang qui comprend un dispositif d'échantillonnage pourvu de plusieurs moyens de transfert selon la figure 2 ;
- les figures 5a à 5e représentent de façon schématique le moyen de transfert du dispositif d'échantillonnage de la figure 2, respectivement de face, en perspective, de profil, de dessus et en coupe transversale, les récipients d'échantillonnage étant en position d'attente ;
- les figures 6a et 6b représentent de façon schématique le moyen de transfert des figures 5 selon une variante de réalisation, respectivement de face et de profil, les récipients étant en position d'attente ; la figure 6c est une vue analogue à la figure 6b dans laquelle un récipient est en position de transfert.

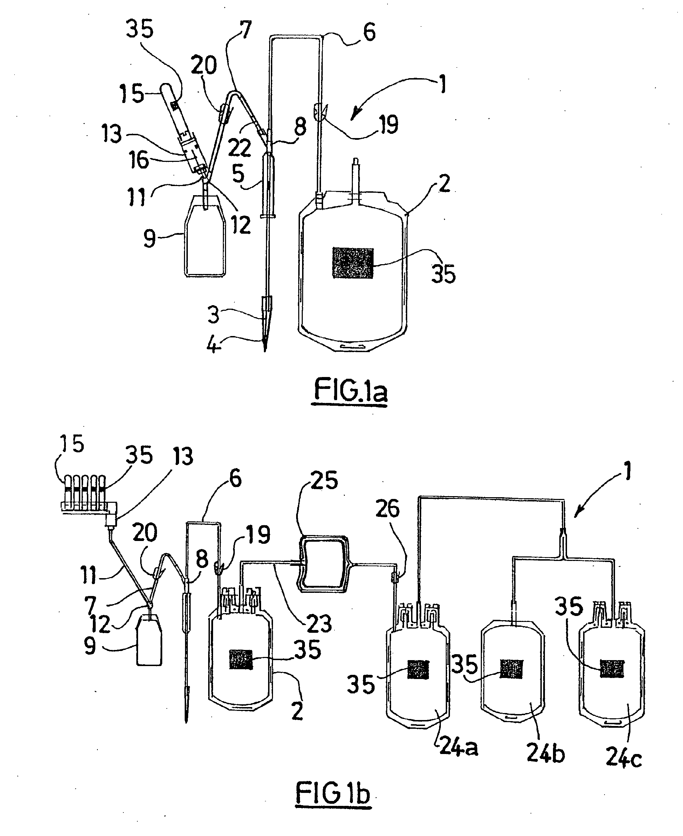

Les figures 1a et 1b représentent un système à poches 1 comprenant des moyens de prélèvement du fluide auprès d'un donneur et au moins une poche de recueil 2 destinée à recevoir le fluide prélevé, notamment du sang.

Les moyens de prélèvement sont constitués notamment d'une aiguille 3 permettant l'accès à la veine du donneur et d'un capuchon 4 protégeant l'aiguille 3. En outre, un protecteur d'aiguille 5 peut être placé de façon coulissante sur une première tubulure 6 mettant en communication la poche de recueil 2 avec les moyens de prélèvement.

Le système à poches 1 comprend en outre un dispositif d'échantillonnage, qui est en communication fluidique avec la poche de recueil 2 par l'intermédiaire de la première 6 et d'une deuxième tubulure 7 reliées au niveau d'un premier connecteur 8 sous la forme d'une jonction trois voies.

Dans les modes de réalisation représentés, le dispositif d'échantillonnage comprend une poche d'échantillonnage 9 qui est connectée à l'extrémité aval de la deuxième tubulure 7. Les termes aval et amont sont définis par rapport au sens de circulation du sang, du dispositif de prélèvement vers les poches et le dispositif d'échantillonnage.

Le dispositif d'échantillonnage comprend en outre un moyen de transfert 10 du fluide, qui est en communication fluidique avec la poche de recueil 2 par l'intermédiaire de la première 6 et de la deuxième tubulure 7, et éventuellement d'une troisième tubulure 11 reliée à la deuxième tubulure 7 au niveau d'un deuxième connecteur 12 sous la forme d'une jonction trois voies.

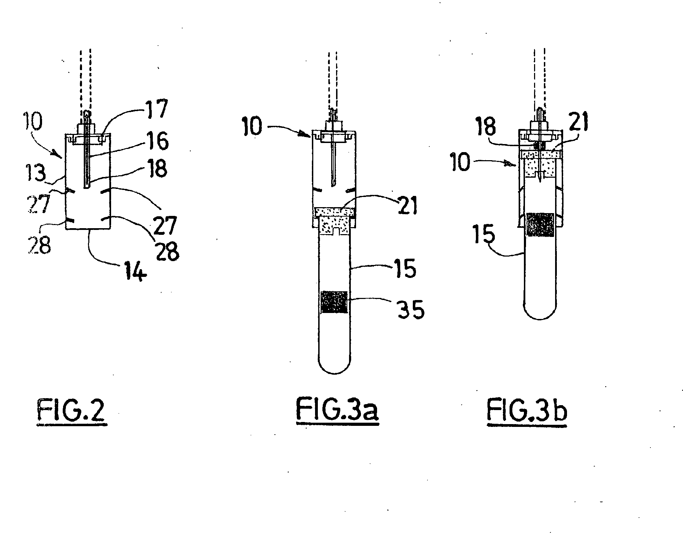

Comme représenté sur la figure 2, le moyen de transfert 10 comprend un guide creux 13, ouvert en partie avant 14 afin de permettre l'introduction d'un récipient d'échantillonnage 15, et une aiguille creuse 16 traversant la partie arrière 17 du guide, de sorte qu'une partie aval de ladite aiguille s'étende à l'intérieur du guide et qu'une partie amont de ladite aiguille 16 s'étende à l'extérieur du guide. Le segment aval de l'aiguille 16 creuse est enfermé dans un fourreau élastique 18. Le segment amont de l'aiguille 16 creuse permet la connexion du moyen de transfert avec le système à poches 1. Un moyen de communication fluidique ou tubulure est alors connectée sur ledit segment amont.

Un premier 19 et un deuxième 20 clamp peuvent être situés respectivement sur la première tubulure 6, en aval du connecteur 8, et sur la deuxième tubulure 7. Les clamps 19, 20 permettent d'orienter le flux du fluide prélevé, soit vers la poche d'échantillonnage 9, le premier clamp 19 est fermé alors que le deuxième clamp 20 est ouvert, soit vers la poche de recueil 2, le deuxième clamp 20 est fermé alors que le premier clamp 19 est ouvert.

Le récipient d'échantillonnage 15 est rempli avec le sang prélevé contenu dans la poche d'échantillonnage 9, lorsque ledit récipient 15 est placé dans la position de transfert, à savoir lorsque l'extrémité aval de l'aiguille 16 est en communication fluidique avec l'intérieur du récipient 15, par perforation d'un élément de fermeture 21 du récipient 15.

Des ouvre-circuits peuvent être prévus au sein du système à poches 1. Notamment un ouvre-circuit 22 peut être situé sur la deuxième tubulure 7 à proximité du premier connecteur 8.

Comme représenté sur la figure 1b, afin de réaliser des étapes de filtration et de séparation ainsi que la déleucocytation des différents constituants du sang, la poche de recueil 2 peut être en communication fluidique, par l'intermédiaire d'une quatrième tubulure 23, avec des poches satellites 24a-c. Un filtre 25 à déleucocyter est situé entre la poche de recueil 2 et une poche satellite 24a. La poche satellite 24a peut être en communication fluidique avec une ou plusieurs autres poches satellites, par exemple la poche satellite 24a peut être en communication fluidique avec deux autres poches satellites 24b,c. Un clamp 26 peut être prévu sur la quatrième tubulure 23 entre la poche de recueil 2 et le filtre 25 à déleucocyter. Selon une réalisation, les poches satellites 24a-c peuvent être pourvues d'un moyen d'identification 35.

Selon un premier mode de réalisation, le moyen de transfert 10 est pourvu d'un moyen d'association du récipient 15 d'échantillonnage, comme représenté sur la figure 2. Le moyen d'association comprend un premier 27 et un deuxième 28 ensemble de saillies réparties longitudinalement sur la surface interne du guide 13, respectivement à proximité de l'aiguille 16 du guide et à proximité de la partie avant 14 du guide 13. Les saillies sont agencées pour être déformables par coulissement du récipient 15 à l'intérieur du guide 13 de sorte à permettre une association réversible du récipient 15 à l'intérieur du guide 13, et un coulissement du récipient 15 à l'intérieur du guide 13 entre une position d'attente (figure 3a), à distance de l'aiguille 16, et la position de transfert (figure 3b).

Comme représenté sur les figures 2, 3a et 3b, les saillies sont souples, notamment élastiques, et sont déformables réversiblement depuis une position inclinée vers l'avant vers une position inclinée vers l'arrière par contact du récipient 15 lors de son coulissement à l'intérieur du guide 13 dans le sens avant-arrière. Lorsque le récipient 15 est retiré du guide 13, les saillies s'inclinent d'arrière en avant de telle sorte que le récipient n'est pas dissocié de son élément de fermeture 21. Dans la réalisation représentée, le récipient 15 d'échantillonnage comprend un élément de fermeture 21 dont le diamètre est supérieur à celui du corps du récipient 15, c'est lors du passage de l'élément de fermeture 21 que les saillies s'inclinent dans un sens ou dans l'autre.

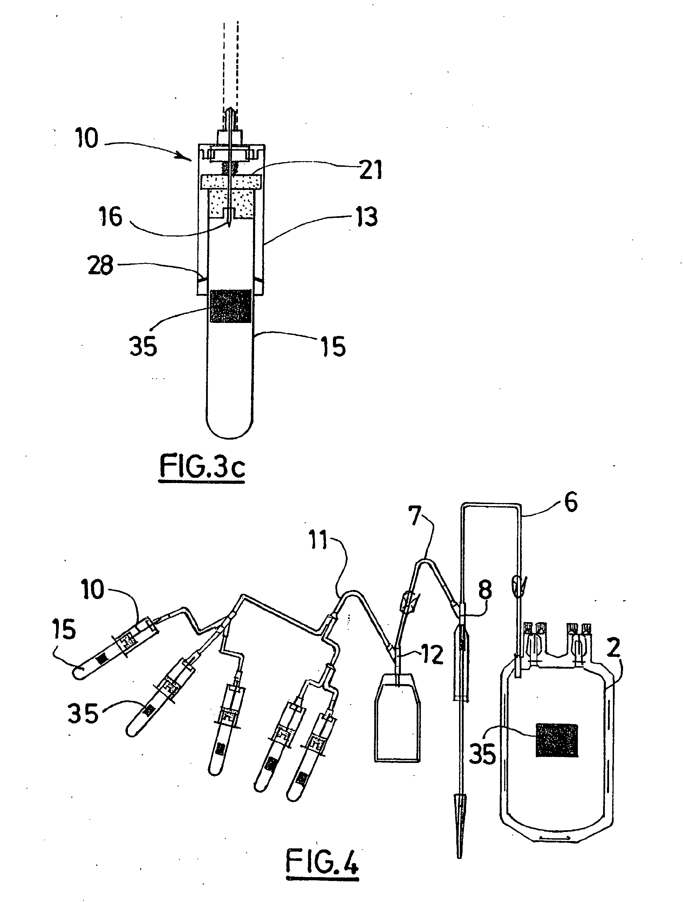

Selon une variante, représentée sur la figure 3c, les saillies du premier ensemble 27 situé à proximité de l'aiguille 16 sont cassables sous l'effet du coulissement du récipient 15 placé dans la position de transfert. La perforation de l'élément de fermeture 21 est ainsi visible, l'utilisateur peut vérifier que la perforation n'a pas eu lieu préalablement à la prise d'échantillons.

Comme représenté sur la figure 4, plusieurs moyens de transfert 10, dans chacun desquels un récipient 15 d'échantillonnage est associé de façon dissociable, peuvent être connectés au système à poches 1 par l'intermédiaire de la deuxième tubulure 7 ou de la troisième tubulure 11, reliée à la deuxième tubulure 7 par le deuxième connecteur 12. Associer de façon dissociable plusieurs récipients 15 à plusieurs moyens de transfert 10 présente des avantages, d'une part, un gain de temps pour la personne chargée du prélèvement puisqu'elle ne doit pas mettre en place le récipient 15 dans le moyen de transfert 10, et, d'autre part, une diminution du risque d'erreur de traçabilité des dons, puisque cela permet de fixer des étiquettes de traçabilité préalablement à la prise d'échantillons, notamment dès la fabrication.

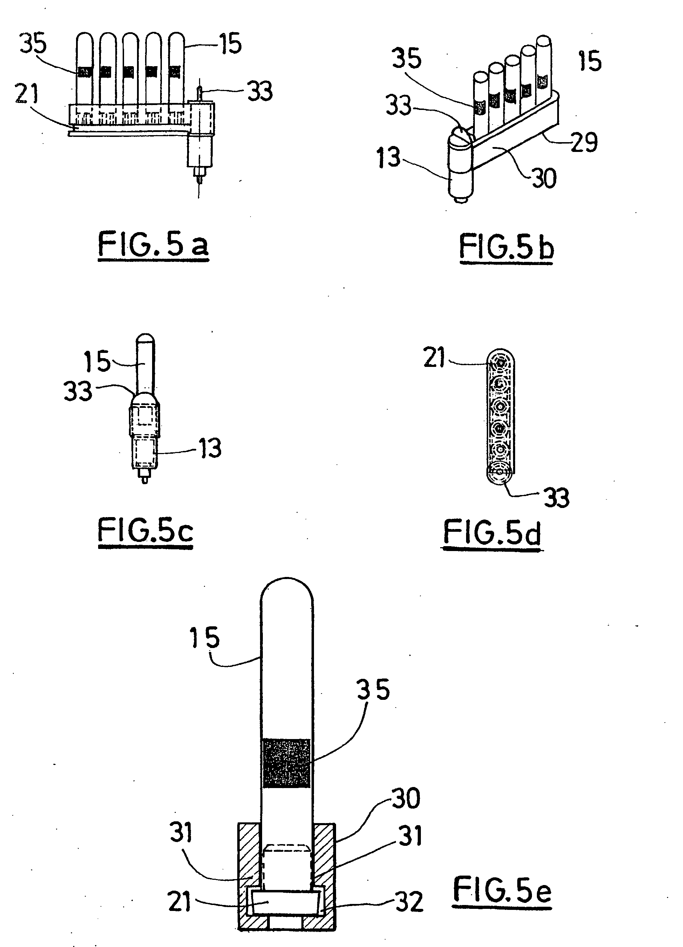

Selon un deuxième mode de réalisation, le moyen d'association est agencé pour permettre le support de plusieurs récipients 15 à distance du guide 13 dans une position d'attente et leur guidage séquentiel dans le guide 13, comme représenté sur les figures 1b et 5a à 5e.

Le moyen d'association et le moyen de transfert 10 sont associés par clipsage, par soudage, ou peuvent être moulés en une seule et même pièce.

Le moyen d'association comprend un boîtier 29 associé au guide 13. Ledit boîtier 29 est pourvu d'une jupe 30 dans laquelle l'élément de fermeture 21 des récipients 15 est introduit pour permettre le coulissement longitudinal des récipients 15 dans le boîtier 29 vers le guide 13. La paroi interne de ladite jupe 30 est munie d'une saillie 31 destinée, par interaction avec les éléments de fermeture 21, à empêcher le retrait transversal des récipients 15 du boîtier 29.

La jupe 30 comprend une extrémité ouverte disposée en regard d'une échancrure formée dans le guide, et une extrémité opposée fermée. Dans l'autre axe, une première extrémité ouverte est disposée en regard d'une autre extrémité qui est ouverte de telle sorte que le corps du ou des récipients 15 s'étende au-delà du boîtier 29.

Lors de la fabrication, les récipients 15 d'échantillonnage sont introduits dans le guide 13 par sa partie avant 14 ouverte de sorte que l'élément de fermeture 21 se situe à la hauteur de la rainure 32 du boîtier 29 afin qu'il puisse être glissé dans celle-ci.

Un capuchon 33 est ensuite placé sur le guide 13 permettant de maintenir les récipients 15 dans le boîtier 29 jusqu'à la prise d'échantillons par l'utilisateur du système 1.

Le boîtier peut être de taille variable de sorte à contenir de deux à dix récipients 15. Le nombre de récipients 15 utilisés varie selon les législations, en France, notamment, cinq récipients 15 sont utilisés pour la réalisation des analyses courantes.

Lors de la prise d'échantillons, la personne chargée du prélèvement retire le capuchon 33 du guide 13, fait glisser les récipients 15 jusqu'au guide 13, puis les introduit de sorte que, par perforation d'un élément de fermeture 21 du récipient 15, l'extrémité aval de l'aiguille 16 est en communication fluidique avec l'intérieur du récipient 15. Après qu'un récipient 15 ait été rempli, l'utilisateur le retire du guide 13. Dans un exemple de réalisation, le capuchon 33 peut être muni d'un élément d'inviolabilité, telle qu'une languette qui est rompue lors de la première ouverture, de sorte à pouvoir identifier la première manipulation du bouchon 33.

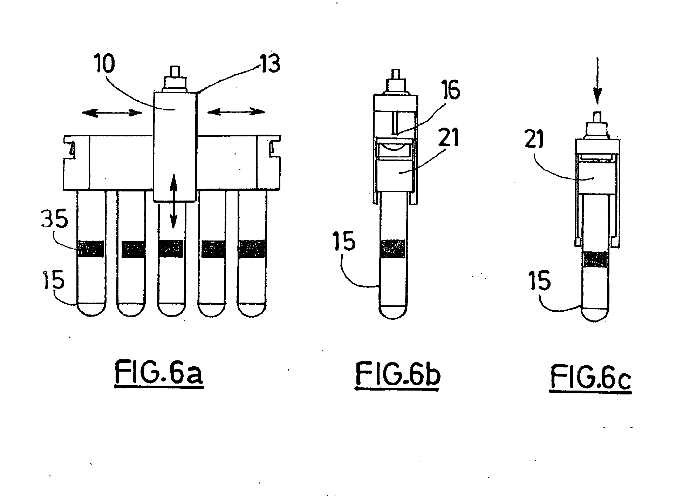

Selon une variante, représentée sur les figures 6a à 6c, le moyen de transfert 10 peut coulisser sur le moyen d'association, de sorte qu'il peut être placé à la hauteur de chaque récipient 15. Lorsque le moyen de transfert 10 est placé à la hauteur d'un récipient 15, l'utilisateur peut alors déplacer ledit moyen de transfert 10 transversalement de sorte que l'élément de fermeture 21 soit perforé par l'aiguille 16. Pour que le moyen de transfert 10 puisse coulisser sur le moyen d'association, deux échancrures opposées sont alors formées dans le guide 13.

Comme représenté sur la figure 1b, le moyen de transfert 10 associant plusieurs récipients 15 d'échantillonnage peut être connecté à un système à poches 1 par l'intermédiaire de la deuxième tubulure 7 et éventuellement de la troisième tubulure 11.

Avec les systèmes à poches pour le prélèvement du sang connus, la personne chargée du prélèvement doit identifier par un marquage, la poche de recueil 2 et le ou les récipients 15 d'échantillons correspondant à un même don.

Selon l'invention, la possibilité d'erreur de traçabilité de ces dons est considérablement réduite puisque le ou les récipients 15 d'échantillonnage et la poche de recueil 2 sont associés de façon dissociable dès la fabrication. En outre, dès la fabrication, la poche de recueil 2 et le ou les récipients 15 d'échantillonnage, ainsi que les éventuelles poches satellites 24a-c, sont pourvus chacun d'un moyen d'identification 35, par exemple au moyen d'une étiquette autocollante à codes barres, qui comprend des informations permettant, après dissociation du récipient d'avec le système à poches 1, d'établir de façon univoque que le récipient 15 d'échantillonnage et la poche de recueil 2, ainsi que les éventuelles poches satellites 24a-c, proviennent du même système à poches 1.

## Revendications

1. Système à poches (1) pour le prélèvement d'un fluide biologique, notamment sanguin, ledit système comprenant un dispositif de prélèvement du fluide qui est en communication fluidique avec au moins une poche de recueil (2) du fluide, et un dispositif d'échantillonnage du fluide à recueillir qui comprend au moins un récipient d'échantillonnage (15), ledit dispositif d'échantillonnage comprenant un moyen de transfert (10) du fluide depuis le système à poches (1) dans le ou les récipient(s) d'échantillonnage (15), ledit moyen de transfert (10) comprenant un guide creux (13) qui est ouvert en partie avant (14) pour permettre l'introduction d'un récipient d'échantillonnage (15), et une aiguille creuse (16) traversant la partie arrière (17) du guide (13) de sorte qu'une partie aval de ladite aiguille s'étende à l'intérieur du guide (13) et qu'une partie amont de ladite aiguille s'étende à l'extérieur du guide (13), ladite aiguille étant en communication fluidique avec le système à poches (1), pour, par translation, permettre la disposition du récipient (15) dans une position de transfert dans laquelle, par perforation d'un élément de fermeture (21) du récipient (15), l'extrémité aval de l'aiguille (16) est en communication fluidique avec l'intérieur du récipient (15), ledit système étant **caractérisé en ce que** le moyen de transfert (10) est pourvu d'un moyen d'association du ou des récipient(s) (15), lesdits moyens de transfert et d'association étant agencés pour permettre le support du ou des récipient(s) (15) dans une position d'attente à distance de l'aiguille (16), et, après transfert, la dissociation du ou des récipients (15) d'avec le système à poches (1).

2. Système selon la revendication 1, **caractérisé en ce que** la poche de recueil (2) est en communication fluidique avec le dispositif de prélèvement par l'intermédiaire d'une première tubulure (6) sur laquelle est connecté le dispositif d'échantillonnage par l'intermédiaire d'une deuxième tubulure (7).

3. Système selon la revendication 2, **caractérisé en ce que** le dispositif d'échantillonnage comprend une poche d'échantillonnage (9) qui est connectée à l'extrémité aval de la deuxième tubulure (7).

4. Système selon l'une quelconque des revendications 1 à 3, **caractérisé en ce que** les récipients (15) comprennent un élément de fermeture (21) dont le diamètre est supérieur à celui du corps du récipient (15).

5. Système selon l'une quelconque des revendications 1 à 4, **caractérisé en ce que** le moyen d'association comprend un premier (27) et un deuxième (28) ensemble de saillies répartis longitudinalement sur la face interne du guide (13), lesdites saillies étant agencés pour être déformables par coulissement du récipient (15) à l'intérieur du guide (13) de sorte à permettre une association réversible du récipient (15) à l'intérieur du guide (13) et un coulissement du récipient (15) à l'intérieur du guide (13) entre une position d'attente à distance de l'aiguille (16) et la position de transfert.

6. Système selon la revendication 5, **caractérisé en ce qu'**au moins certaines saillies sont souples, notamment élastiques, et sont déformables réversiblement depuis une position inclinée vers l'arrière vers une position inclinée vers l'avant par contact du récipient (15) lors de son coulissement à l'intérieur du guide (13) dans le sens arrière-avant, respectivement avant-arrière.

7. Système selon la revendication 5 ou 6, **caractérisé en ce que** les saillies du premier ensemble (27) qui est situé à proximité de l'aiguille (16) sont cassables sous l'effet de la déformation.

8. Système selon l'une quelconque des revendications 2 à 7, **caractérisé en ce qu'**il comprend plusieurs moyens de transfert (10) dans chacun desquels un récipient (15) est associé de façon dissociable, lesdits moyens étant connectés au système (1) par l'intermédiaire de la deuxième tubulure (7) ou d'une troisième tubulure (11).

9. Système selon l'une quelconque des revendications 1 à 8, **caractérisé en ce que** le moyen d'association est agencé pour permettre le support de plusieurs récipients (15) à distance du guide (13) dans une position d'attente et leur guidage séquentiel dans le guide (13).

10. Système selon la revendication 9 lorsqu'elle dépend de la revendication 4, **caractérisé en ce que** le moyen d'association comprend un boîtier (29) associé au guide (13), ledit boîtier étant pourvu d'une jupe (30) dans laquelle les extrémités fermées des récipients (15) sont introduites pour permettre le coulissement longitudinal des récipients (15) dans le boîtier (29) vers le guide (13), la paroi interne de ladite jupe étant munie d'une saillie (31) destinée, par interaction avec les éléments de fermeture (21), à empêcher le retrait transversal des récipients (15) du boîtier (29).

11. Système selon la revendication 10, **caractérisé en ce que** la jupe (30) comprend une extrémité ouverte disposée en regard d'une échancrure formée dans le guide (13), et une extrémité opposée fermée.

12. Système selon l'une quelconque des revendications 9 à 11, **caractérisé en ce que** le guide (13) est muni d'un capuchon (33) qui est pourvu d'un élément d'inviolabilité.

13. Système selon l'une quelconque des revendications 9 à 12, **caractérisé en ce qu'**il comprend un moyen de transfert (10) associant plusieurs récipients (15), ledit moyen étant connecté au système par l'intermédiaire de la deuxième tubulure (7) ou d'une troisième tubulure (11).

14. Système selon l'une quelconque des revendications 1 à 13, **caractérisé en ce que** la poche de recueil (2) et le ou les récipient(s) (15) d'échantillonnage sont pourvus chacun d'un moyen d'identification (35) qui comprend des informations permettant, après dissociation du récipient (15) d'avec le système à poches (1), d'établir de façon univoque que le récipient d'échantillonnage (15) et la poche de recueil (2) proviennent du même système à poches (1).

15. Système selon l'une quelconque des revendications 1 à 14, **caractérisé en ce que** la poche de recueil (2) est en communication fluidique, par l'intermédiaire d'une quatrième tubulure (23), avec des poches satellites (24a-c), lesdites poches satellites étant pourvues d'un moyen d'identification (35).

## Claims

1. A bag system (1) for sampling a biological fluid, more particularly blood, said system including a fluid sampling device which is in fluidic communication with at least a fluid collection bag (2), and a sampling device for the fluid to be collected, which includes at least a sampling container (15), said sampling device including means (10) for transferring the fluid from the bag system (1) into the sampling container/containers (15), said transfer means (10) including a hollow guide (13) which is open on the front (14) to enable the introduction of a sampling container (15), and a hollow needle (16) going through the rear part (17) of the guide (13), so that a downstream part of said needle extends inside the guide (13), and an upstream part of said needle extends outside the guide (13), with said needle being in fluidic communication with the bag system (1), to enable, by translation, the positioning of the container (15) in a transfer position, wherein the downstream end of the needle (16) is in fluidic communication with the inside of the container (15), through the perforation of a closure element (21) of the container (15), with said system being **characterized in that** the transfer means (10) is provided with means for associating the container/containers (15), said transfer and association means being so arranged to enable the support of the container/containers (15) in a stand-by position away from the needle (16), and, after the transfer, the detachment of the container/containers (15) from the bag system (1).

2. A system according to claim 1, **characterized in that** the collection bag (2) is in fluidic communication with the sampling device through a first tubing (6) to which the sampling device is connected through a second tubing (7).

3. A system according to claim 2, **characterized in that** the sampling device includes a sampling bag (9) which is connected to the downstream end of the second tubing (7).

4. A system according to any one of claims 1 to 3, **characterized in that** the containers (15) include a closure element (21) the diameter of which is greater than that of the container (15) body.

5. A system according to any one of claims 1 to 4, **characterized in that** the association means include a first (27) and a second (28) set of protrusions longitudinally distributed on the inner face of the guide (13), said protrusions being arranged to be deformable by sliding the container (15) inside the guide (13) so as to enable a reversible association of the container (15) inside the guide (13) and sliding the container (15) inside the guide (13) between a stand-by position away from the needle (16) and the transfer position.

6. A system according to claim 5, **characterized in that** at least some protrusions are flexible, more particularly resilient and are reversibly deformable from a rearward inclined position to a frontward inclined position through the contact of the container (15) upon the sliding thereof inside the guide (13) in the rear-front, respectively front-rear direction.

7. A system according to claim 5 or 6, **characterized in that** the protrusions of the first set (27) which is positioned close to the needle (16) can break because of the deformation.

8. A system according to any one of claims 2 to 7, **characterized in that** it includes several transfer means (10) wherein each of which a container (15) is detachably associated, with said means being connected to the system (1) through the second tubing (7) or a third tubing (7).

9. A system according to any one of claims 1 to 8, **characterized in that** the association means is so arranged as to enable the support of several containers (15) away from the guide (13) in a stand-by position and the sequential guiding thereof in the guide (13).

10. A system according to claim 9, when it depends on claim 4, **characterized in that** the association means includes a housing (29) associated with the guide (13), said housing being provided with a skirt (30), wherein the closed ends of the containers (15) are introduced to enable the longitudinal sliding of the containers (15) in the housing (29) towards the guide (13), with the inner wall of said skirt being provided with a protrusion (31) intended to prevent the transversal removal of the containers (15) from the housing (29) through an interaction with the closure elements (21).

11. A system according to claim 10, **characterized in that** the skirt (30) includes an open end positioned opposite a notch provided in the guide (13), and an opposite closed end.

12. A system according to any one of claims 9 to 11, **characterized in that** the guide (13) is provided with a cap (33) which is provided with a tamper resistance element.

13. A system according to any one of claims 9 to 12, **characterized in that** it includes transfer means (10) associating several containers (15), with said means being connected to the system through the second tubing (7) or a third tubing (11).

14. A system according to any one of claims 1 to 13, **characterized in that** the collection bag (2) and the sampling container/containers (15) is/are each provided with identification means (35) which includes information making it possible, following the detachment of the container (15) from the bag system (1), to unambiguously establish that the sampling container (15) and the collection bag (2) come from the same bag system (1).

15. A system according to any one of claims 1 to 14, **characterized in that** the collection bag (2) is in fluidic communication, through a fourth tubing (23), with satellite bags (24a-c), with said satellite bags being provided with identification means (35).

## Patentansprüche

1. Beutelsystem (1) für die Entnahme einer biologischen Flüssigkeit, insbesondere Blut, wobei das besagte System eine Entnahmevorrichtung der Flüssigkeit umfaßt, die strömungstechnisch in Verbindung mit mindestens einem Auffangbeutel (2) der Flüssigkeit steht, und eine Probenahme-Vorrichtung der aufzufangenden Flüssigkeit, die mindestens einen Probenahme-Behälter (15) umfaßt, wobei die besagte Probenahme-Vorrichtung ein Transfermittel (10) der Flüssigkeit ab dem Beutelsystem (1) in den oder die Probenahme-Behälter (15) umfaßt, wobei das besagte Transfermittel (10) einen hohlen Führer (13) umfaßt, der am vorderen Teil (14) offen ist, damit ein Probenahme-Behälter (15) eingeführt werden kann, und eine Hohlnadel (16), die den rückwärtigen Teil (17) des Führers (13) durchquert, so daß sich ein unterer Teil der besagten Nadel im Innern des Führers (13) erstreckt und sich ein oberer Teil der besagten Nadel außerhalb des Führers (13) erstreckt, wobei die besagte Nadel strömungstechnisch in Verbindung mit dem Beutelsystem (1) steht, um durch Translation die Anordnung des Behälters (15) in einer Transferposition zu ermöglichen, in der durch Perforation eines Verschlußelements (21) des Behälters (15) das untere Ende der Nadel (16) strömungstechnisch in Verbindung mit dem Innern des Behälters (15) steht, wobei das besagte System **dadurch gekennzeichnet ist, daß** das Transfermittel (10) mit einem Verbindungsmittel des oder der Behälter(s) (15) versehen ist, wobei die besagten Transfer- und Verbindungsmittel gestaltet sind, um das Tragen des oder der Behälter (15) in einer von der Nadel entfernten Warteposition (16) und nach dem Transfer die Trennung des oder der Behälter(s) (15) vom Beutelsystem (1) zu ermöglichen.

2. System nach Anspruch 1, **dadurch gekennzeichnet, daß** der Sammelbeutel (2) strömungstechnisch in Verbindung mit der Entnahmevorrichtung steht und dies vermittels eines ersten Stutzens (6), an den die Probenahme-Vorrichtung vermittels eines zweiten Stutzens (7) angeschlossen ist.

3. System nach Anspruch 2, **dadurch gekennzeichnet, daß** die Probenahme-Vorrichtung einen Probenahmebeutel (9) umfaßt, der an das untere Ende des zweiten Stutzens (7) angeschlossen ist.

4. System nach einem beliebigen der vorstehenden Ansprüche 1 bis 3, **dadurch gekennzeichnet, daß** die Behälter (15) ein Verschlußelement (21) umfassen, dessen Durchmesser größer als derjenige des Körpers des Behälters (15) ist.

5. System nach einem beliebigen der vorstehenden Ansprüche 1 bis 4, **dadurch gekennzeichnet, daß** das Vereinigungsmittel einen ersten (27) und einen zweiten (28) Satz Vorsprünge umfaßt, die längs über die Innenseite des Führers (13) verteilt sind, wobei die besagten Vorsprünge so gestaltet sind, daß, sie durch Verschieben des Behälters (15) im Innern des Führers (13) verformbar sind, so daß eine umkehrbare Vereinigung des Behälters (15) im Innern des Führers (13) und ein Verschieben des Behälters (15) im Innern des Führers (13) zwischen einer von der Nadel entfernten Warteposition (16) und der Transferposition möglich ist.

6. System nach Anspruch 5, **dadurch gekennzeichnet, daß** zumindest manche Vorsprünge weich und insbesondere elastisch und umkehrbar verformbar sind ab einer nach hinten geneigten Position zu einer nach vorne geneigten Position, und zwar durch Kontakt des Behälters (15) bei seinem Verschieben im Innern des Führers (13) in der Richtung von hinten nach vorne beziehungsweise von vorne nach hinten.

7. System nach Anspruch 5 oder 6, **dadurch gekennzeichnet, daß** die Vorsprünge des ersten Satzes (27), der sich in der Nähe der Nadel (16) befindet, unter Einwirkung der Verformung zerbrechlich sind.

8. System nach einem beliebigen der vorstehenden Ansprüche 2 bis 7, **dadurch gekennzeichnet, daß** es mehrere Transfermittel (10) umfaßt, wobei in jedem davon ein Behälter (15) trennbar vereint ist, wobei die besagten Mittel vermittels des zweiten Stutzens (7) oder eines dritten Stutzens (11) an das System (1) angeschlossen sind.

9. System nach einem beliebigen der vorstehenden Ansprüche 1 bis 8, **dadurch gekennzeichnet, daß** das Vereinigungsmittel gestaltet ist, um das Tragen mehrerer Behälter (15) entfernt vom Führer (13) in einer Warteposition und ihre Ablaufführung im Führer (13) zu ermöglichen.

10. System nach Anspruch 9, wenn er vom Anspruch 4 abhängt, **dadurch gekennzeichnet, daß** das Vereinigungsmittel einen mit dem Führer (13) verbundenen Kasten (29) umfaßt, wobei der besagte Kasten mit einer Schürze (30) versehen ist, in der die verschlossenen Enden der Behälter (15) eingesetzt sind, damit das Verschieben der Behälter (15) in Längsrichtung im Kasten (29) zum Führer (13) möglich ist, wobei die Innenwand der besagten Schürze mit einem Vorsprung (31) versehen ist, der dazu bestimmt ist, durch Zusammenwirken mit den Verschlußelementen (21) das Herausnehmen in Querrichtung der Behälter (15) aus dem Kasten (29) zu verhindern.

11. System nach Anspruch 10, **dadurch gekennzeichnet, daß** die Schürze (30) ein offenes Ende umfaßt, das gegenüber einem im Führer (13) gebildeten Ausschnitt angeordnet ist, und ein geschlossenes entgegengesetztes Ende.

12. System nach einem beliebigen der vorstehenden Ansprüche 9 bis 11, **dadurch gekennzeichnet, daß** der Führer (13) mit einer Kappe (33) versehen ist, die mit einem Unversehrtheitselement versehen ist.

13. System nach einem beliebigen der vorstehenden Ansprüche 9 bis 12, **dadurch gekennzeichnet, daß** es ein Transfermittel (10) umfaßt, das mehrere Behälter (15) verbindet, wobei das besagte Mittel vermittels des zweiten Stutzens (7) oder eines dritten Stutzens (11) an das System angeschlossen ist.

14. System nach einem beliebigen der vorstehenden Ansprüche 1 bis 13, **dadurch gekennzeichnet, daß** der Auffangbeutel (2) und der oder die Probenahme-Behälter (15) jeweils mit einem Erkennungsmittel (35) versehen sind, das Informationen umfaßt, die ermöglichen, nach dem Trennen des Behälters (15) vom Beutelsystem (1) unzweideutig klarzustellen, daß der Probenahme-Behälter (15) und der Auffangbeutel (2) vom gleichen Beutelsystem (1) stammen.

15. System nach einem beliebigen der vorstehenden Ansprüche 9 bis 14, **dadurch gekennzeichnet, daß** der Auffangbeutel (2) vermittels eines vierten Stutzens (23) strömungstechnisch in Verbindung mit Satellitenbeuteln (24a-c) steht, wobei die besagten Satellitenbeutel mit einem Erkennungsmittel (35) versehen sind.
